(19) Europäisches Patentamt
European Patent Office
Office européen des brevets

(11) EP 0 603 322 B1

(12) **FASCICULE DE BREVET EUROPEEN**

(45) Date de publication et mention de la délivrance du brevet: **11.12.1996 Bulletin 1996/50**

(21) Numéro de dépôt: **92920522.7**

(22) Date de dépôt: **11.09.1992**

(51) Int Cl.6: **C07D 209/08**, A61K 7/13

(86) Numéro de dépôt international: **PCT/FR92/00856**

(87) Numéro de publication internationale: **WO 93/05018 (18.03.1993 Gazette 1993/08)**

(54) **COMPOSITION TINCTORIALE A BASE DE DERIVES D'HYDROXYINDOLINE**

FÄRBEMITTEL AUF BASIS VON HYDROXYINDOLINDERIVATEN

DYEING COMPOSITION BASED ON HYDROXYINDOLINE DERIVATIVES

(84) Etats contractants désignés: **AT BE CH DE DK ES FR GB GR IT LI NL SE**

(30) Priorité: **12.09.1991 FR 9111281**

(43) Date de publication de la demande: **29.06.1994 Bulletin 1994/26**

(73) Titulaire: **L'OREAL**
**75008 Paris (FR)**

(72) Inventeurs:
- **LAGRANGE, Alain**
**F-77700 Serris (FR)**
- **LUPPI, Bernadette**
**F-93270 Sevran (FR)**
- **JUNINO, Alex**
**F-93180 Livry-Gargan (FR)**

(74) Mandataire: **Casalonga, Axel et al**
**BUREAU D.A. CASALONGA - JOSSE**
**Morassistrasse 8**
**80469 München (DE)**

(56) Documents cités:
**EP-A- 462 857**
**WO-A-91/17739**
**DE-A- 1 916 139**
**FR-A- 2 636 237**
**GB-A- 2 187 456**
**US-A- 4 013 404**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 603 322 B1

Printed by Jouve, 75001 PARIS (FR)

## Description

La présente invention est relative à l'utilisation pour la teinture des fibres kératiniques, en particulier des fibres kératiniques humaines telles que les cheveux, de dérivés d'hydroxyindoline, aux compositions tinctoriales, aux procédés de teinture mettant en oeuvre ces composés, ainsi qu'à des nouveaux dérivés d'hydroxyindoline.

On a déjà proposé dans le passé de teindre les cheveux en utilisant comme coupleurs, certaines monohydroxyindolines ou monoaminoindolines, notamment dans le brevet français n° 2 008 797; le brevet US-A-4 013 404 décrit des mono- ou diamino indolines ou des monohydroxyindolines comme base d'oxydation ou comme coupleurs, utilisés en teinture d'oxydation des cheveux.

WO 91/17739 décrit l'utilisation d'indolines hydroxylées ou alcoxylées, en positions 5 et 6 comme précurseurs de colorants d'oxydation ou comme coupleurs. Le seul composé illustré dans ce document est le bromhydrate de la 5,6-dihydroxyindoline. La demande de brevet EP-A-4 62 857 concerne la 5,6-dihydroxyindoline et certains de ses dérivés N-alkylés. Ces deux derniers documents, antérieurs à la présente demande, n'ont été publiés qu'après sa date de dépôt.

On connaît, par ailleurs, les colorants de la famille des indoles, en particulier le 5,6-dihydroxyindole pour leur utilisation en teinture des fibres kératiniques telles que les cheveux humains, notamment par les brevets français FR-A-1 133 594 et 1 166 172.

La demanderesse vient de découvrir que des dérivés d'hydroxyindoline, en particulier sous forme salifiée, présentaient par rapport au 5,6-dihydroxyindole connu, des propriétés particulièrement remarquables en ce qui concerne la stabilité au stockage dans les milieux habituellement utilisés en teinture des fibres kératiniques.

Ces composés présentent, en outre, une bonne solubilité dans l'eau par rapport au 5,6-dihydroxyindole.

Les inventeurs ont constaté par ailleurs que ces composés s'oxydaient particulièrement facilement en solution alcaline et pouvaient être utilisés en teinture capillaire sans éventuellement mettre en oeuvre un agent oxydant, ce qui permet d'obtenir une gamme variée de nuances plus ou moins intenses.

L'invention a donc pour objet l'utilisation pour la teinture des fibres kératiniques, en particulier des cheveux, de dérivés d'hydroxyindoline et de leurs sels de formule (I) définie ci-après, à titre de seuls précurseurs de colorants.

Un autre objet de l'invention est constitué par les compositions tinctoriales destinées à la teinture des fibres kératiniques et en particulier des cheveux humains, contenant à titre de seul précurseur de colorant au moins un dérivé d'hydroxyindoline de formule (I) définie ci-après.

L'invention a également pour objet des procédés de teinture mettant en oeuvre ces composés.

D'autres objets de l'invention apparaîtront à la lecture de la description et des exemples qui suivent.

Les dérivés d'hydroxyindoline utilisés pour la teinture des fibres kératiniques et en particulier des fibres kératiniques humaines telles que les cheveux, conformément à l'invention, sont essentiellement caractérisés par le fait qu'ils répondent à la formule :

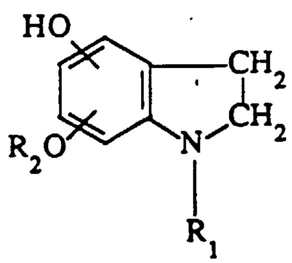

(I)

dans laquelle :

$R_1$ représente un atome d'hydrogène ou un groupement alkyle en $C_1$-$C_4$;

$R_2$ représente un atome d'hydrogène ou un groupement alkyle en $C_1$-$C_4$; les groupes OH et $-OR_2$ occupant les positions 4,5,6 ou 7 du cycle benzénique, sous réserve que lorsqu'ils sont en position 5 et 6, $R_2$ représente un alkyle en $C_1$-$C_4$;

ainsi que les sels d'addition d'acides de ces composés.

On peut citer les chlorhydrates et les bromhydrates comme sels de ces composés.

Les composés préférés utilisés conformément à l'invention sont choisis parmi :

- la 7-méthoxy 6-hydroxyindoline
- la 6,7-dihydroxyindoline

- la 5-hydroxy 4-méthoxyindoline
- la 4,5-dihydroxyindoline
- la 5-méthoxy 6-hydroxyindoline
- la 7-hydroxy 6-méthoxyindoline
- la 7-méthoxy 4-hydroxyindoline
- la 4-hydroxy 5-méthoxyindoline
- la 5-hydroxy 6-méthoxyindoline
- la 4,7-dihydroxyindoline.

Les bromhydrates des composés ci-dessus sont particulièrement préférés.

Les composés particuliers de formule (IA), dans laquelle $R_2$ représente un alkyle, peuvent être préparés par débenzylation de l'alcoxybenzyloxyindoline correspondante, en présence d'acide bromhydrique à une température inférieure ou égale à 40°C, selon le schéma réactionnel :

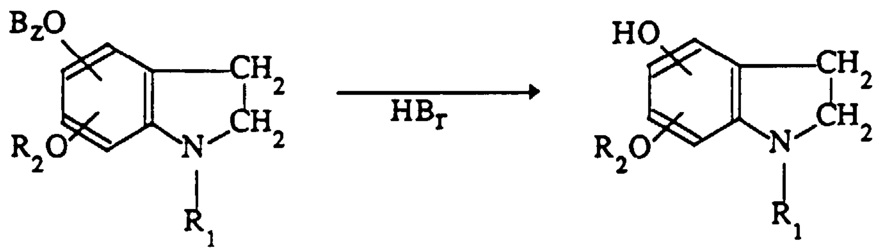


(II) (IA)

SCHEMA A

Les N-alkylbenzyloxyalcoxyindolines de formule (II) dans laquelle $R_1$ désigne un alkyle en $C_1$-$C_4$, peuvent être préparées par alkylation classique de la benzyloxyalcoxyindoline à l'aide d'halogénure d'alkyle ou de sulfate de dialkyle.

Les N-alkylhydroxyalcoxyindolines de formule (IA) peuvent être préparées par alkylation directe de l'hydroxyalcoxyindoline correspondante.

Les composés particuliers de formule (IB) dans laquelle $R_2$ représente un atome d'hydrogène peuvent être préparés :

- soit par déalcoxylation des composés (IA) par l'acide bromhydrique à une température supérieure ou égale à 80°C;
- soit par déméthylation de la diméthoxyindoline correspondante, par l'acide bromhydrique à une température supérieure ou égale à 80°C selon le schéma réactionnel :

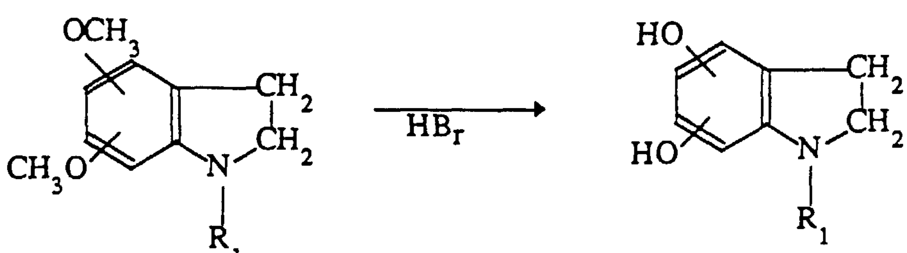


(III) (IB)

SCHEMA B

Les diméthoxy N-alkylindolines de formule (III) dans laquelle $R_1$ désigne alkyle en $C_1$-$C_4$, peuvent être préparées :

- soit par alkylation classique de la diméthoxyindoline à l'aide d'halogénure d'alkyle ou de sulfate de dialkyle;
- ou bien par réduction des dialcoxyindoles par le borohydrure de sodium en présence d'un acide carboxylique selon la méthode de GRIBBLL décrite dans J.A.C.S. - 1974, 96, 7812.

Les dihydroxy N-alkylindolines de formule (IB) peuvent être préparées par alkylation directe de la dihydroxyindoline à l'exclusion de la 5,6-dihydroxyindoline.

Les bromhydrates des dérivés d'hydroxyindoline de formule (I) sont des composés nouveaux et constituent un objet de l'invention. Ils sont préparés selon les procédés indiqués ci-dessus.

Ces composés sont particulièrement stables au stockage dans les conditions habituelles de stockage des compositions tinctoriales pour fibres kératiniques.

Un autre objet de l'invention est constitué par les deux composés nouveaux :

- la 6-hydroxy 7-méthoxyindoline
- la 6,7-dihydroxyindoline

ainsi que leurs sels.

Les dérivés d'hydroxyindoline de formule (I) définie ci-dessus, sont généralement mis en oeuvre à l'aide de compositions qui constituent un autre objet de l'invention.

Les compositions tinctoriales destinées à être utilisées pour la teinture des fibres kératiniques et en particulier les fibres kératiniques humaines telles que les cheveux, conformes à l'invention, sont caractérisées par le fait qu'elles contiennent à titre de seul précurseur de colorant, dans un milieu approprié pour la teinture, au moins un dérivé d'hydroxyindoline répondant à la formule (I) définie ci-dessus.

La quantité de dérivé d'hydroxyindoline de formule (I) utilisée dans la composition, est comprise généralement dans des proportions de 0,01 à 8% en poids par rapport au poids total de la composition et de préférence de 0,03 à 5% en poids.

Ces compositions peuvent se présenter sous des formes diverses, notamment sous forme de lotions plus ou moins épaissies, de crèmes, de mousses et de gels, éventuellement conditionnées sous forme d'aérosols.

Elles peuvent également constituer un élément d'un agent de teinture à plusieurs composants disposé dans un dispositif à plusieurs compartiments ou kit de teinture.

Le milieu approprié pour la teinture est de préférence un milieu aqueux qui doit être cosmétiquement acceptable lorsque les compositions sont destinées à être utilisées pour la teinture des cheveux humains vivants. Ce milieu aqueux peut être constitué par de l'eau ou un mélange eau/solvant(s).

Le pH des compositions est compris entre 3 et 12.

Les solvants sont choisis parmi les solvants organiques et préférentiellement parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

Les solvants particulièrement préférés sont l'alcool éthylique, le propylèneglycol et le monobutyléther d'éthylèneglycol.

Les composés conformes à l'invention présentent l'avantage de pouvoir être utilisés dans un milieu essentiellement aqueux.

Il est également possible d'utiliser un milieu constitué de solvants anhydres choisis parmi les solvants définis ci-dessus. La composition est dans ce cas soit mélangée au moment de l'emploi avec un milieu aqueux, soit appliquée sur les fibres kératiniques mouillées au préalable par une composition aqueuse.

On appelle, conformément à l'invention, un milieu solvant anhydre, un milieu contenant moins de 1% d'eau.

Lorsque le milieu approprié pour la teinture est constitué par un mélange eau/solvant(s), les solvants sont utilisés dans des concentrations comprises entre 0,5 et 75% en poids par rapport au poids total de la composition, et de préférence dans des proportions inférieures à 20% en poids.

Les compositions conformes à l'invention peuvent contenir des adjuvants habituellement utilisés pour la teinture des fibres kératiniques et en particulier des adjuvants cosmétiquement acceptables lorsque ces compositions sont appliquées pour la teinture des cheveux humains vivants.

Ces compositions peuvent contenir notamment des amides gras dans des proportions préférentielles de 0,05 à 10% en poids, des agents tensio-actifs anioniques, cationiques, non ioniques, amphotères ou leurs mélanges présents de préférence dans des proportions comprises entre 0,1 et 50% en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les agents épaississants sont choisis parmi l'alginate de sodium, la gomme arabique, la gomme de guar, les hétérobiopolysaccharides, tels que la gomme de xanthane, les scléroglucanes, les dérivés de cellulose, tels que la méthylcellulose, l'hydroxyéthylcellulose, l'hydroxypropylméthylcellulose, le sel de sodium de la carboxyméthylcellulose et les polymères d'acide acrylique réticulés de préférence.

On peut également utiliser les agents épaississants minéraux, tels que la bentonite.

Ces épaississants sont utilisés seuls ou en mélange et sont présents de préférence dans des proportions comprises entre 0,1 et 5% en poids par rapport au poids total de la composition et avantageusement entre 0,5 et 3% en poids.

Les agents alcalinisants utilisables dans les compositions peuvent être en particulier des amines, telles que les alcanolamines, des alkylamines, des hydroxydes ou carbonates alcalins ou d'ammonium.

Les agents d'acidification utilisables dans ces compositions peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux, en cosmétique.

Lorsque les compositions sont utilisées sous forme de mousse, elles peuvent être conditionnées sous pression et dans un dispositif aérosol en présence d'un agent propulseur et d'au moins un générateur de mousse.

Les agents générateurs de mousse peuvent être des polymères moussants anioniques, cationiques, non ioniques, amphotères ou leurs mélanges ou des agents tensio-actifs du type de ceux définis ci-dessus.

Le procédé de teinture des fibres kératiniques, en particulier des fibres kératiniques humaines, qui constitue un autre objet de l'invention, est essentiellement caractérisé par le fait qu'il consiste à appliquer sur ces fibres une composition (A) définie ci-dessus et contenant dans un milieu approprié pour la teinture, au moins un dérivé d'hydroxyindoline de formule (I) définie ci-dessus, à maintenir la composition au contact des fibres pendant un temps suffisant pour développer la coloration, soit à l'air, soit à l'aide d'un système oxydant, à rincer et éventuellement à laver les fibres ainsi teintes.

Selon une première forme de mise en oeuvre de l'invention, la coloration des fibres peut être effectuée sans addition d'un agent oxydant extérieur, au seul contact de l'air.

Selon une autre forme de réalisation, la couleur est révélée à l'aide d'un système oxydant chimique choisi parmi :

(i) des ions iodure et du peroxyde d'hydrogène, la composition (A) contenant le dérivé d'hydroxyindoline de formule (I) comprenant dans ce cas en plus, soit des ions iodure, soit du peroxyde d'hydrogène, et l'application de la composition (A) est précédée ou suivie par l'application d'une composition (B) qui contient dans un milieu approprié pour la teinture, soit :

(a) du peroxyde d'hydrogène à un pH compris entre 2 et 12 et de préférence entre 2 et 7, lorsque la composition (A) contient des ions iodure, soit :
(b) des ions iodure à un pH compris entre 3 et 11 lorsque la composition (A) contient du peroxyde d'hydrogène;

(ii) des nitrites, l'application de la composition (A) contenant le dérivé d'hydroxyindoline de formule (I) étant suivie par l'application d'une composition aqueuse (B) présentant un pH acide, la composition (A) ou la composition (B) contenant au moins un nitrite;
(iii) des oxydants choisis parmi le peroxyde d'hydrogène, l'acide periodique et ses sels hydrosolubles, l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium, les chlorites alcalins; ces oxydants étant présents dans la composition (A) contenant le dérivé d'hydroxyindoline de formule (I) ou appliqués simultanément ou séquentiellement au moyen d'une composition (B) les contenant dans un milieu approprié pour la teinture;
(iv) des anions d'un métal choisis parmi les permanganates ou bichromates, ces oxydants étant appliqués au moyen d'une composition (B) aqueuse, à un pH de 2 à 10, avant l'application de la composition (A);
(v) des sels métalliques des groupes 3 à 8 du tableau périodique, ces sels métalliques étant appliqués au moyen d'une composition (B) les contenant dans un milieu approprié pour la teinture, la composition (B) étant appliquée préalablement ou postérieurement à l'application de la composition (A);
(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A) contenant le dérivé d'hydroxyindoline de formule (I);
(vii) un dérivé quinonique choisi parmi les ortho- ou parabenzoquinones, les ortho- ou parabenzoquinones monoimines ou diimines, les 1,2- ou 1,4-naphtoquinones, les ortho- ou parabenzoquinones sulfonimides, les $\alpha,\omega$-alkylène-bis-1,4-benzoquinones ou les 1,2 ou 1,4-naphtoquinones monoimines ou diimines au moyen d'une composition (B) les contenant dans un milieu approprié pour la teinture, le dérivé d'hydroxyindoline de formule (I) et les dérivés quinoniques étant choisis de façon à ce que la différence de potentiel d'oxydoréduction $\Delta E$ entre le potentiel d'oxydoréduction Ei du dérivé d'hydroxyindoline de formule (I) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts};$$

la composition (B) étant appliquée préalablement ou postérieurement à l'application de la composition (A) contenant le dérivé d'hydroxyindoline de formule (I).

Selon une forme préférée de l'invention, l'application des compositions (A) et (B) est séparée par une étape de rinçage à l'eau.

A titre de dérivés quinoniques utilisables dans ce procédé, on peut citer :

- la 1,4-benzoquinone
- la 2-méthoxy 1,4-benzoquinone
- la 2-méthyl 1,4-benzoquinone
- la 2,6-diméthyl 1,4-benzoquinone
- la 2,3,5-trichloro 6-méthyl 1,4-benzoquinone
- la 2-acétylamino 1,4-benzoquinone
- la 2-acétylamino 3,5-diméthyl 1,4-benzoquinone
- la 2,6-diméthyl 5-acétylamino 1,4-benzoquinone
- la 2-chloro 1,4-benzoquinone
- la tétrachloro 1,2-benzoquinone
- la 2,3-diméthoxy 1,4-benzoquinone
- la 2-β-carboxyéthoxy 1,4-benzoquinone
- la 2-méthoxyméthyl 1,4-benzoquinone
- la 2-β-hydroxyéthyl 1,4-benzoquinone
- la 2-β-hydroxyéthylthio 1,4-benzoquinone
- la 2,5-bis-β-hydroxyéthylthio 1,4-benzoquinone
- la 2-β,γ-dihydroxypropylthio 1,4-benzoquinone
- la 2-β-carboxyéthylthio 1,4-benzoquinone
- la 2-carboxyméthyl 1,4-benzoquinone
- la 2-β-hydroxyéthylthio 6-méthyl 1,4-benzoquinone
- la 2-méthoxycarbonyl 3-méthoxy 1,4-benzoquinone
- la 2-méthoxycarbonyl 1,4-benzoquinone
- la 2-méthylthio 1,4-benzoquinone
- la 2-diméthylamino 1,4-benzoquinone
- la 2-acétylamino 5-méthoxy 1,4-benzoquinone
- la 2-(β-hydroxyéthylthio)méthyl 1,4-benzoquinone
- la 2-(méthylthio)méthyl 1,4-benzoquinone
- la 4,5-diméthoxy 1,2-benzoquinone
- la 4-méthyl 5-chloro 1,2-benzoquinone
- la 4,5-diméthyl 1,2-benzoquinone
- la 2,3-diméthyl 1,4-benzoquinone
- la 2-β-hydroxyéthoxy 1,4-benzoquinone
- la N-méthyl sulfonyl 1,4-benzoquinone monoimine
- la N-phényl sulfonyl 1,4-benzoquinone monoimine
- la 1,4-naphtoquinone
- la 1,2-naphtoquinone
- l'acide 1,2-naphtoquinone 4-sulfonique
- la 2,3-dichloro 1,4-naphtoquinone
- la N-2,6-trichloro 1,4-benzoquinone imine.

Selon une première variante du procédé de teinture mettant en oeuvre des systèmes oxydants, on applique sur les matières kératiniques une composition (A) contenant dans un milieu approprié pour la teinture, au moins un composé de formule (I) en association avec des ions iodure, l'application de la composition (A) étant précédée ou suivie par l'application d'une composition (B) qui contient. dans un milieu approprié pour la teinture, du peroxyde d'hydrogène.

Ce procédé peut également être mis en oeuvre en appliquant sur les fibres kératiniques au moins une composition (A) contenant dans un milieu approprié pour la teinture, le composé de formule (I) en association avec du peroxyde d'hydrogène, ayant un pH compris entre 2 et 7 et de préférence entre 3,5 et 7, l'application de la composition (A) étant précédée ou suivie par l'application d'une composition (B) qui contient dans un milieu approprié pour la teinture, des ions iodure.

L'ion iodure dans cette variante du procédé, est choisi de préférence parmi les iodures de métaux alcalins, alcalino-terreux ou d'ammonium. L'iodure est plus particulièrement constitué par l'iodure de potassium.

Les ions iodure sont présents dans les compositions (A) ou (B) dans des proportions comprises généralement entre 0,007 et 4% en poids exprimées en ions $I^-$ et de préférence entre 0,08 et 1,5% en poids par rapport au poids total de la composition (A) ou (B).

Selon une seconde variante, ce procédé peut être mis en oeuvre en utilisant comme agent oxydant pour révéler la coloration, un nitrite. Les nitrites plus particulièrement utilisables conformément à l'invention, sont :

- des nitrites de métaux alcalins, alcalino-terreux ou d'ammonium ou de tout autre cation cosmétiquement acceptable lorsqu'il est utilisé pour la teinture des cheveux humains vivants;
- des dérivés organiques des nitrites tels que par exemple le nitrite d'amyle;
- des vecteurs de nitrites, c'est-à-dire des composés qui par transformation forment des nitrites du type défini ci-dessus.

Les nitrites particulièrement préférés sont les nitrites de sodium, de potassium ou d'ammonium.

Cette variante du procédé est mise en oeuvre en appliquant sur les matières kératiniques, la composition (A) à base du composé de formule (I) définie ci-dessus, puis une composition aqueuse acide (B), la composition (A) ou (B) contenant au moins un nitrite.

Les nitrites sont généralement utilisés dans des proportions comprises entre 0,02 et 1 mole/litre.

Selon une troisième variante de ce procédé, les oxydants sont choisis parmi le peroxyde d'hydrogène, la chloramine T, la chloramine B, l'acide periodique et ses sels hydrosolubles, l'hypochlorite de sodium, le ferricyanure de potassium, l'oxyde d'argent, le réactif de Fenton, l'oxyde de plomb (IV), le sulfate de cesium, le persulfate d'ammonium. Ces agents sont de préférence appliqués sur les fibres au moyen d'une composition (B) et après l'application de la composition (A).

Ces agents oxydants sont présents dans des proportions suffisantes pour développer une coloration et de préférence dans des proportions comprises entre 0,004 mole et 0,7 mole, en particulier entre 0,01 mole et 0,04 mole pour 100 g de composition.

Selon une quatrième variante de ce procédé, on applique dans un premier temps sur les fibres kératiniques, une composition contenant dans un milieu approprié pour la teinture, à un pH compris entre 2 et 10, un anion d'un métal ayant une bonne affinité pour la kératine et ayant un potentiel d'oxydoréduction supérieur à celui des composés de formule (I). Cet anion est choisi de préférence parmi les permanganates ou les bichromates et plus particulièrement le permanganate de potassium et le bichromate de sodium.

Ces anions métalliques sont généralement utilisés à des molalités en anions supérieures à $10^{-3}$ mole/1000 g jusqu'à de préférence de 1 mole/1000 g.

Dans un second temps, on applique une composition contenant dans un milieu approprié pour la teinture, à un pH compris entre 4 et 10, un composé répondant à la formule (I) définie ci-dessus.

Les compositions contenant les anions ne doivent pas contenir d'agents organiques ayant un effet réducteur sur les anions.

Selon une cinquième variante de l'invention, on met en oeuvre des catalyseurs d'oxydation choisis parmi les sels métalliques tels que les sels de manganèse, de cobalt, de fer, de cuivre et d'argent.

A titre d'exemple, on peut utiliser le sulfate de manganèse, le lactate de manganèse, le chlorure de cobalt, le chlorure ferrique, le chlorure cuivrique, le nitrate d'argent ammoniacal.

Les sels préférés sont les sels de cuivre. Ces sels sont utilisés dans des proportions de 0,01 à 2% exprimées en ions métalliques par rapport au poids total de la composition mise en oeuvre et contenant ces sels.

Selon cette variante, on met les fibres kératiniques, en particulier les cheveux, en contact avec une composition (B) contenant dans un milieu approprié pour la teinture, le sel métallique, avant ou après l'application de la composition (A) contenant le composé de formule (I) et on rince de préférence entre les deux étapes.

La forme de réalisation préférée consiste à appliquer un sel cuivrique dans un premier temps et la composition (A) contenant le dérivé d'hydroxyindoline de formule (I), dans un deuxième temps.

On peut faire suivre cette teinture après rinçage, de l'application d'une solution de peroxyde d'hydrogène pour éventuellement éclaircir la couleur obtenue.

Selon une sixième variante, on utilise des sels de terres rares. Les sels de terres rares utilisables conformément à l'invention sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents dans des proportions comprises entre 0,1 et 8% en poids par rapport au poids total de la composition.

On utilise de préférence les sels de Cérium $Ce^{3+}$ et $Ce^{4+}$ sous forme de sulfates et de chlorures.

Selon une septième variante, la composition contenant le dérivé quinonique est appliquée avant ou après la composition (A) contenant le composé de formule (I).

A titre de dérivé quinonique préféré, on peut citer la 1,4-benzoquinone et la 2-β-hydroxyéthylthio 1,4-benzoquinone.

La concentration en dérivés quinoniques est de préférence comprise entre 0,005 et 1 mole/litre dans la composition (B). Le pH de la composition (B) est compris entre 2 et 10 et de préférence inférieur à 7.

Lorsqu'on met en oeuvre des compositions à base de peroxyde d'hydrogène dans les différents procédés décrits ci-dessus, la teneur en peroxyde d'hydrogène est généralement comprise entre 1 et 40 volumes et de préférence entre 2 et 10 volumes et plus particulièrement entre 3 et 10 volumes.

L'invention a également pour objet un agent de coloration des fibres kératiniques et en particulier des fibres kératiniques humaines, à plusieurs composants, destiné notamment à être utilisé dans la mise en oeuvre du procédé de teinture défini ci-dessus et utilisant un système oxydant. Dans ce cas, l'agent de teinture comprend au moins deux composants dont un premier est constitué par la composition (A) définie ci-dessus et contenant le dérivé d'hydroxyindoline de formule (I) et l'autre composant est constitué par l'une des compositions (B) également définie ci-dessus.

Les composants (A) et (B) respectifs sont choisis selon les différentes variantes du procédé exposées ci-dessus.

L'invention a également pour objet un dispositif à plusieurs compartiments ou encore "kit de teinture" ou "nécessaire de teinture", comportant tous les composants destinés à être appliqués dans une même teinture sur les fibres kératiniques en application unique ou successives avec ou sans prémélange comme mentionné ci-dessus.

De tels dispositifs sont connus en eux-mêmes et peuvent comporter un premier compartiment contenant la composition (A) contenant le dérivé d'hydroxyindoline de formule (I) dans un milieu approprié pour la teinture, et dans un second compartiment une composition (B) du type défini ci-dessus et contenant l'agent oxydant.

Les dispositifs à plusieurs compartiments utilisables conformément à l'invention, peuvent être équipés des moyens de mélanges au moment de l'emploi et leur contenu peut être conditionné sous atmosphère inerte.

Lorsque le milieu contenant le dérivé d'hydroxyindoline de formule (I) est anhydre, on peut prévoir un troisième compartiment contenant un milieu aqueux approprié pour la teinture et destiné à être mélangé tout juste avant l'emploi avec la composition du premier compartiment.

Le dérivé d'hydroxyindoline de formule (I), les compositions et le procédé conformes à l'invention, peuvent être mis en oeuvre pour teindre des cheveux naturels ou déjà teints, permanentés ou non, défrisés ou non, ou des cheveux fortement ou légèrement décolorés et éventuellement permanentés.

Il est également possible de les utiliser pour la teinture de la fourrure ou de la laine.

Les exemples sont destinés à illustrer l'invention sans pour autant présenter un caractère limitatif.

EXEMPLE DE PREPARATION 1

SYNTHESE DU BROMHYDRATE DU 6-HYDROXY-7-METHOXY INDOLINE

A) *Synthèse de la 6-benzyloxy-7-méthoxyindoline*

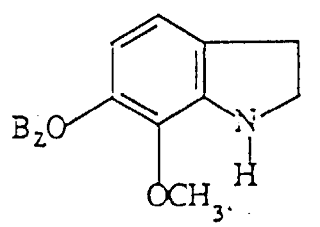

A une solution refroidie sous azote de 7 g de 6-benzyloxy-7-metoxy indole dans 50 ml de diméthoxyéthane, on ajoute 2,4 g de borohydrure de sodium par spatulée. On coule, goutte à goutte, 12 ml d'acide trifluoro-acétique. Après 1 heure à la température ambiante, on ajoute 40 ml de soude à 15% et on verse dans 200 ml d'eau. On extrait trois fois avec 100 ml d'acétate d'éthyle. Les phases organiques sont réunies, lavées à l'eau, séchées et évaporées à sec pour donner 7 g de résidu. On chromatographie le résidu sur silice avec un gradient du mélange Heptane/Acétate d'éthyle (95/5 à 80/20). On obtient 3,1 g d'un liquide légèrement jaune que l'on utilise directement pour l'étape suivante.

On observe une tache unique en chromatographie sur couche mince.

Rf = 0,41 Heptane/Acétate d'éthyle 50/50.

B) *Synthèse du bromhydrate de6-hydroxy-7-méthoxyindoline*

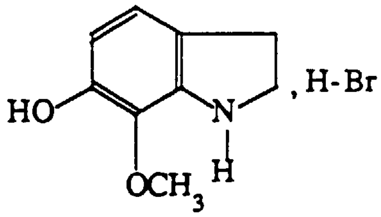

L'indoline de l'exemple 1A) (3 g) est mise en suspension dans 25 ml d'acide bromhydrique à 47%. Après 1 heure d'agitation à 40°C, on dilue avec 25 ml d'eau permutée, puis on évapore sous vide. Le résidu vert sombre est remis en solution dans de l'eau. On y ajoute 10 g de charbon végétal et on chauffe pendant 30 minutes au bain marie bouillant. On filtre sur célite et on évapore à sec. La poudre beige est recristallisée dans un mélange éthanol/éther éthylique. On obtient 1,6 g de cristaux beiges ayant comme caractéristique :

| Analyse élémentaire pour $C_9H_{12}Br\ N\ O_2$ | | | | | |
|---|---|---|---|---|---|
| | C | H | Br | N | O |
| Calculé | 43,92 | 4,91 | 32,47 | 5,69 | 13,00 |
| Trouvé | 43,70 | 5,05 | 32,19 | 5,60 | 12,86 |

EXEMPLE DE PREPARATION 2

**SYNTHESE DU BROMHYDRATE DE 6,7-DIHYDROXY INDOLINE**

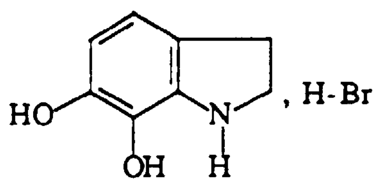

On mélange 15 g de 6-benzyloxy-7-méthoxyindoline (exemple 1A) et 100 ml d'acide bromhydrique à 47%. Ce mélange est porté au reflux de l'acide pendant 1 heure. Par refroidissement, il y a formation d'un précipité. Celui-ci est filtré puis remis en solution à chaud dans de l'eau. On y ajoute 10 g de charbon végétal et on maintient au bain-marie bouillant pendant 30 minutes. On filtre sur célite et on évapore le filtrat. Le solide obtenu est lavé à l'éther éthylique puis à l'acétone. Le solide est redissous dans de l'eau puis filtré et évaporé pour conduire à 3,4 g d'un solide beige.

Le spectre RMN du proton est conforme à la structure attendue.

EXEMPLE 1

**COMPOSITION (A)**

| | | |
|---|---|---|
| - Bromhydrate de 4-hydroxy 5-méthoxy-indoline | 1 g | |
| - Iodure de potassium | 0,8 g | |
| - Alcool éthylique à 96° | 10 g | |
| - Hydroxyéthylcellulose vendue sous la dénomination NATROSOL 250 HHR par la Société AQUALON | 1 g | |
| - Alkyléther de glucoside vendu à 60% de MA sous la dénomination TRITON CG 110 par la Société ROHM & HAAS | 5 g | MA |
| - Lauryléther sulfate de sodium | 0,2 g | MA |
| - pH spontané = 4,9 | | |

(suite)

| | | |
|---|---|---|
| - Eau déminéralisée qsp | 100 g | |

**COMPOSITION (B)**

Solution d'eau oxygénée à 12,5 volumes.

On applique la composition (A) sur les cheveux gris permanentés pendant 15 minutes à température ambiante. On rince, puis on applique la composition (B) pendant 5 minutes. Après rinçage et séchage, les cheveux sont teints en blond foncé doré irisé.

EXEMPLE 2

**COMPOSITION (A)**

| | |
|---|---|
| - Bromhydrate de 6-hydroxy 7-méthoxy-indoline | 1 g |
| - Lauryléther sulfate de sodium oxyéthyléné à 2 moles d'oxyde d'éthylène | 15 g |
| - Monobutyléther d'éthylèneglycol | 10 g |
| - Eau déminéralisée qsp | 100 g |
| - pH spontané = 3,1 | |

**COMPOSITION (B)**

| | |
|---|---|
| - Periodate de sodium | 5 g |
| - HCl qs pH=3,0 | |
| - Eau déminéralisée qsp | 100 g |

On applique la composition (A) sur des cheveux gris à 90% de blancs pendant 15 minutes. Après rinçage, on applique la composition (B) pendant 10 minutes.

Les cheveux sont rincés, puis séchés. ns sont teints en blond clair naturel cendré.

EXEMPLE 3

| | |
|---|---|
| - Bromhydrate de 6,7-dihydroxyindoline | 0,5 g |
| - Alcool éthylique à 96° | 10 g |
| - Hydroxypropylcellulose vendue sous la dénomination KLUCEL G par la Société AQUALON | 2 g |
| -Alkyl($C_8$-$C_{10}$)polyglucoside en solution aqueuse à 60% tamponnée, vendu sous la dénomination TRITON CG 110-60 par la Société ROHM & HAAS | 3,5 g |
| - Nonylphénol oxyéthyléné à 9 moles d'oxyde d'éthylène | 0,1 g |
| - Acide tartrique | 0,3 g |
| - Triéthanolamine | 3,75 g |
| - pH = 8,1 | |
| - Eau déminéralisée qsp | 100 g |

On applique cette composition sur des cheveux gris à 90% de blancs pendant 10 minutes. Après rinçage et séchage, les cheveux sont teints en blond clair doré.

Cette couleur s'intensifie au cours d'applications successives.

EXEMPLE 4

**COMPOSITION (A)**

| | | |
|---|---|---|
| - Bromhydrate de 4-hydroxy 5-méthoxy-indoline | 1 g | |
| - Alcool éthylique à 96° | 10 g | |
| - Hydroxyéthylcellulose vendue sous la dénomination NATROSOL 250 HHR par la Société AQUALON | 1 g | |
| - Alkyléther de glucoside vendu à 60% de MA sous la dénomination TRITON CG 110 par la Société ROHM & HAAS | 5 g | MA |
| - Eau déminéralisée qsp | 100 g | |
| - pH spontané = 4,8 | | |

**COMPOSITION (B)**

| | |
|---|---|
| - Eau oxygénée à 20 volumes | 66 ml |
| - Solution aqueuse d'ammoniaque à 3,4% qsp | 100 ml |

On applique la composition (A) sur des cheveux gris permanentés pendant 10 minutes. Après rinçage, on applique la composition (B) pendant 20 minutes.

Les cheveux sont alors rincés puis séchés.

Ils sont teints en blond clair doré mat.

EXEMPLE 5

**COMPOSITION (A)**

| | |
|---|---|
| - Bromhydrate de 4,5-dihydroxyindoline | 1 g |
| - Alcool éthylique à 96° | 10 g |
| - Propylèneglycol | 2 g |
| - Nonylphénol oxyéthylé à 9 moles d'oxyde d'éthylène | 4 g |
| - Eau déminéralisée qsp | 100 g |
| - pH spontané = 3,9 | |

**COMPOSITION (B)**

| | |
|---|---|
| - Nitrite de sodium | 2 g |
| - Eau déminéralisée qsp | 100 g |
| - HCl qs pH=3,8 | |

On applique la composition (A) sur des cheveux gris à 90% de blancs pendant 20 minutes. Après rinçage, on applique la composition (B) pendant 5 minutes. Les cheveux sont alors rincés puis séchés. Ils sont teints en blond cendré doré mat.

EXEMPLE 6

**COMPOSITION (A)**

| | |
|---|---|
| - Sulfate de cuivre, $5H_2O$ | 1 g |
| - Lauryléther sulfate de sodium oxyéthyléné à 2 moles d'oxyde d'éthylène | 15 g |
| - Hydroxyéthylcellulose | 2,4 g |
| - Monoéthanolamine qs pH=9,5 | |
| - Eau déminéralisée qsp | 100 g |

**COMPOSITION (B)**

| | |
|---|---|
| - Bromhydrate de 4,5-dihydroxyindoline | 1 g |
| - Alcool éthylique à 96° | 10 g |
| - Lauryléther sulfate de sodium oxyéthyléné à 2 moles d'oxyde d'éthylène | 15 g |
| - Eau déminéralisée qsp | 100 g |
| - pH spontané = 3,9 | |

On applique la composition (A) sur des cheveux gris permanentés pendant 5 minutes. Après rinçage, on applique la composition (B) pendant 10 minutes. Les cheveux sont rincés puis séchés. Ils sont teints en cendré bleu profond.

EXEMPLE 7

**COMPOSITION (A)**

| | |
|---|---|
| - Sulfate de cérium | 1 g |
| - Eau déminéralisée qsp | 100 g |
| - pH spontané = 1,6 | |

**COMPOSITION (B)**

| | |
|---|---|
| - Bromhydrate de 4,5-dihydroxyindoline | 1 g |
| - Monobutyléther d'éthylèneglycol | 10 g |
| - Nonylphénol oxyéthyléné à 9 moles d'oxyde d'éthylène | 15 g |
| - Eau déminéralisée qsp | 100 g |
| - pH spontané = 4,0 | |

On applique la composition (A) sur des cheveux gris permanentés pendant 10 minutes. On rince et on applique la composition (B) pendant 10 minutes.

Après rinçage et séchage, les cheveux sont teints en blond foncé cendré.

EXEMPLE 8

**COMPOSITION (A)**

| | |
|---|---|
| - Bromhydrate de 5-méthoxy 6-hydroxyindoline | 2 g |
| - Alcool éthylique à 96° | 8 g |
| - Eau déminéralisée qsp | 100 g |

(suite)

| | |
|---|---|
| - pH spontané = 3,0 | |

**COMPOSITION (B)**

| | |
|---|---|
| - Chlorite de sodium | 4 g |
| - Eau déminéralisée qsp | 100 g |
| - pH spontané = 10,1 | |

On mélange, juste avant l'emploi, poids pour poids, la composition (A) et la composition (B). Ce mélange est appliqué pendant 20 minutes sur des cheveux gris à 90% de blancs. Après rinçage et séchage, les cheveux sont teints en blond foncé cendré.

**Revendications**

**1.** Utilisation pour la teinture des fibres kératiniques, en particulier des fibres kératiniques humaines, d'un dérivé d'hydroxyindoline de formule :

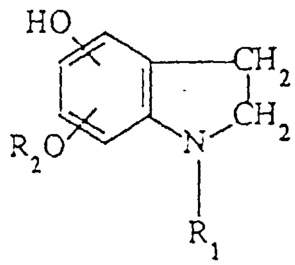

(I)

dans laquelle :

R1 représente un atome d'hydrogène ou un radical alkyle en C1-C4 ;
R2 représente un atome d'hydrogène ou un radical alkyle en C1-C4 ;
OH et -OR2 occupent les positions 4, 5, 6 ou 7 du cycle benzénique sous réserve que lorsqu'ils occupent les positions 5 et 6, R2 désigne un alkyle en C1-C4 ;
ainsi que leurs sels d'addition d'acides, à titre de seul précurseur de colorant.

**2.** Utilisation selon la revendication 1, caractérisée par le fait que les sels sont choisis parmi les chlorhydrates ou les bromhydrates.

**3.** Utilisation selon la revendication 1, caractérisée par le fait que le dérivé d'hydroxyindoline est choisi parmi la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 7-hydroxy 6-méthoxyindoline, la 7-méthoxy 4-hydroxyindoline, la 4-hydroxy 5-méthoxyindoline, la 5-hydroxy 6-méthoxyindoline, la 4,7-dihydroxyindoline ou leurs sels d'addition d'acides.

**4.** Composition tinctoriale destinée à être utilisée pour la teinture des fibres kératiniques, caractérisée par le fait qu'elle contient à titre de seul précurseur de colorant, dans un milieu approprié pour la teinture, au moins un dérivé d'hydroxyindoline de formule (I) tel que défini dans l'une quelconque des revendications 1 à 3.

**5.** Composition selon la revendication 4, caractérisée par le fait que le dérivé d'hydroxyindoline de formule (I) est présent dans la composition dans des proportions comprises entre 0,01 et 8% en poids par rapport au poids total de la composition.

6. Composition selon la revendication 4 ou 5, caractérisée par le fait que le milieu approprié pour la teinture est un milieu aqueux constitué par de l'eau ou un mélange eau/solvant(s).

7. Composition selon la revendication 6, caractérisée par le fait que les solvants sont choisis parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

8. Composition selon l'une quelconque des revendications 4 à 7, caractérisée par le fait que la composition contient des amides gras, des agents tensio-actifs anioniques, cationiques, non-ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques ou leurs mélanges.

9. Composition selon l'une quelconque des revendications 4 à 8, caractérisée par le fait que le pH de la composition est compris entre 3 et 12.

10. Procédé de teinture des fibres kératiniques, en particulier des fibres kératiniques humaines, caractérisé par le fait que l'on applique sur ces fibres au moins une composition telle que définie dans l'une quelconque des revendications 4 à 9, que l'on maintient cette composition au contact des fibres pendant un temps suffisant pour développer une coloration, soit à l'air, soit à l'aide d'un système oxydant et qu'on rince ensuite.

11. Procédé selon la revendication 10, caractérisé par le fait que l'on laisse la coloration se développer au contact de l'air sans additionner d'agent oxydant extérieur.

12. Procédé selon la revendication 10, caractérisé par le fait que l'on applique sur les fibres une composition (A) contenant dans un milieu approprié pour la teinture, au moins un dérivé d'hydroxyindoline de formule (I) tel que défini dans l'une quelconque des revendications 1 à 3, la couleur étant révélée à l'aide d'un système oxydant chimique constitué par :

    (i) des ions iodure et du peroxyde d'hydrogène, la composition (A) contenant dans ce cas, en plus, soit (a) des ions iodure, soit (b) du peroxyde d'hydrogène et l'application de la composition (A) étant précédée ou suivie par l'application d'une composition (B) qui contient dans un milieu approprié pour la teinture, soit :

        (a) du peroxyde d'hydrogène à un pH compris entre 2 et 12 lorsque la composition (A) contient des ions iodure, soit :
        (b) des ions iodure à un pH compris entre 3 et 11 lorsque la composition (A) contient du peroxyde d'hydrogène;

    (ni) des nitrites, l'application de la composition (A) étant suivie par l'application d'une composition aqueuse (B) présentant un pH acide, la composition (A) ou la composition (B) contenant au moins un nitrite;
    (iii) des oxydants choisis parmi le peroxyde d'hydrogène, l'acide periodique et ses sels hydrosolubles, l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium, les chlorites alcalins; ces oxydants étant présents dans la composition (A) contenant le dérivé d'hydroxyindoline de formule (I) ou appliqués simultanément ou séquentiellement au moyen d'une composition (B) les contenant dans un milieu approprié pour la teinture;
    (iv) des anions d'un métal choisis parmi les permanganates ou bichromates, ces oxydants étant appliqués au moyen d'une composition (B) aqueuse, à un pH de 2 à 10, avant l'application de la composition (A);
    (v) des sels métalliques des groupes 3 à 8 du tableau périodique, ces sels métalliques étant appliqués au moyen d'une composition (B) les contenant dans un milieu approprié pour la teinture, la composition (B) étant appliquée préalablement ou postérieurement à l'application de la composition (A);
    (vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A) contenant le dérivé d'hydroxyindoline de formule (I);
    (vii) un dérivé quinonique choisi parmi les ortho- ou parabenzoquinones, les ortho- ou parabenzoquinones monoimines ou diimines, les 1,2- ou 1,4-naphtoquinones, les ortho- ou parabenzoquinones sulfonimides, les $\alpha,\omega$-alkylène-bis-1,4-benzoquinones ou les 1,2 ou 1,4-naphtoquinones monoimines ou diimines au moyen

d'une composition (B) les contenant dans un milieu approprié pour la teinture, le dérivé d'hydroxyindoline de formule (I) et les dérivés quinoniques étant choisis de façon à ce que la différence de potentiel d'oxydoréduction ΔE entre le potentiel d'oxydoréduction Ei du dérivé d'hydroxyindoline de formule (I) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts;}$$

la composition (B) étant appliquée préalablement ou postérieurement à l'application de la composition (A) contenant le dérivé d'hydroxyindoline de formule (I).

**13.** Procédé selon la revendication 12, caractérisé par le fait que l'on applique sur les matières kératiniques une composition (A) contenant dans un milieu approprié pour la teinture, au moins un dérivé d'hydroxyindoline de formule (I) en association avec des ions iodure, l'application de la composition (A) étant précédée ou suivie par l'application d'une composition (B) qui contient dans un milieu approprié pour la teinture, du peroxyde d'hydrogène.

**14.** Procédé selon la revendication 12, caractérisé par le fait que l'on applique sur les fibres kératiniques, au moins une composition (A) contenant dans un milieu approprié pour la teinture, un dérivé d'hydroxyindoline de formule (I) en association avec du peroxyde d'hydrogène et ayant un pH compris entre 2 et 7, l'application de la composition (A) étant précédée ou suivie par l'application d'une composition (B) qui contient dans un milieu approprié pour la teinture, des ions iodure.

**15.** Procédé selon la revendication 13 ou 14, caractérisé par le fait que les ions iodure sont présents dans la composition (A) ou (B) dans des proportions comprises entre 0,007 et 4% en poids exprimées en ions $I^-$ par rapport au poids total de la composition (A) ou (B).

**16.** Procédé selon la revendication 12, caractérisé par le fait que l'on applique sur les matières kératiniques une composition (A) contenant le dérivé d'hydroxyindoline de formule (I) et que l'on applique ensuite une composition aqueuse acide (B), la composition (A) ou la composition (B) contenant au moins un nitrite choisi parmi les nitrites de métaux alcalins, alcalino-terreux ou d'ammonium ou de tout autre cation cosmétiquement acceptable, un dérivé organique de nitrite et des vecteurs de nitrite générant un nitrite du type défini ci-dessus, ces nitrites étant présents dans des proportions comprises entre 0,02 et 1 mole/litre.

**17.** Procédé selon la revendication 12, caractérisé par le fait que l'on applique sur les fibres kératiniques une composition (B) contenant dans un milieu approprié pour la teinture, à un pH compris entre 2 et 10, un anion d'un métal ayant une bonne affinité pour la kératine choisi parmi les permanganates ou les bichromates et que dans un second temps, on applique une composition (A) contenant dans un milieu approprié pour la teinture, à un pH compris entre 4 et 10, un dérivé d'hydroxyindoline répondant à la formule (I), ces permanganates ou bichromates étant utilisés dans des molalités en anions supérieures à $10^{-3}$ mole/1000 g
et que les compositions ne contiennent pas d'agent organique ayant un effet réducteur sur les anions.

**18.** Procédé selon la revendication 12, caractérisé par le fait que l'on applique sur les fibres kératiniques une composition (A) contenant dans un milieu approprié pour la teinture, un dérivé d'hydroxyindoline de formule (I) et que l'on applique avant ou après la composition (A) une composition (B) contenant dans un milieu approprié pour la teinture, un sel métallique choisi parmi les sels de manganèse, de cobalt, de fer, de cuivre et d'argent, ces sels de métaux étant utilisés dans des proportions comprises entre 0,01 et 2% en poids exprimé en ions métalliques par rapport au poids total de la composition.

**19.** Procédé selon la revendication 12, caractérisé par le fait que l'on applique une composition (A) contenant dans un milieu approprié pour la teinture, au moins un dérivé d'hydroxyindoline de formule (I) et qu'avant ou après cette composition, on applique une composition (B) contenant dans un milieu approprié pour la teinture, un sel de terres rares choisi parmi les sels de Cérium, de Lanthane, d'Europium, de Gadolinium, d'Ytterbium, de Dysprosium, ces sels de terres rares étant présents dans des proportions comprises entre 0,1 et 8% en poids par rapport au poids total de la composition.

**20.** Procédé selon la revendication 12, caractérisé par le fait que l'on applique une composition (A) contenant dans

un milieu approprié pour la teinture, au moins un dérivé d'hydroxyindoline de formule (I), et qu'avant ou après cette composition, on applique une composition (B) contenant dans un milieu approprié un dérivé quinonique choisi parmi la 1,4-benzoquinone et la 2-β-hydroxyéthylthio 1,4-benzoquinone, ce dérivé quinonique étant présent dans des proportions comprises entre 0,005 et 1 mole/litre.

**21.** Procédé selon les revendications 12 à 15, caractérisé par le fait que lorsqu'on utilise comme moyen oxydant une composition à base de peroxyde d'hydrogène, la teneur en peroxyde d'hydrogène dans la composition est comprise entre 1 et 40 volumes.

**22.** Agent de teinture des fibres kératiniques, en particulier des fibres kératiniques humaines, à plusieurs composants, caractérisé par le fait qu'il comprend un premier composant constitué par une composition (A) contenant un dérivé d'hydroxyindoline de formule (I) définie dans l'une quelconque des revendications 12 à 20, et un second composant constitué par l'une des compositions (B) définie dans l'une quelconque des revendications 12 à 21.

**23.** Dispositif à plusieurs compartiments ou "kit de teinture", caractérisé par le fait que ces différents compartiments contiennent les différents composants de l'agent de teinture défini dans la revendication 22.

**24.** Composés constitués par les bromhydrates des dérivés d'hydroxyindoline de formule (I), tels que définis dans l'une quelconque des revendications 1 à 3.

**25.** 6-hydroxy 7-méthoxyindoline et ses sels d'addition d'acides.

**26.** 6,7-dihydroxyindoline et ses sels d'addition d'acides.

**27.** Composition selon la revendication 5, caractérisée par le fait que le dérivé d'hydroxyindoline de formule (I) est présent dans la composition dans des proportions comprises entre 0,03 et 5% en poids par rapport au poids total de la composition.

**28.** Procédé selon la revendication 17, caractérisé en ce que les permanganates ou bichromates sont utilisés dans des molalités en anion allant jusqu'à une mole pour 1000 g.

**Patentansprüche**

**1.** Zur Färbung keratinischer Fasern, insbesondere menschlicher keratinischer Fasern, vorgesehene Verwendung eines Hydroxyindolinderivats der Formel:

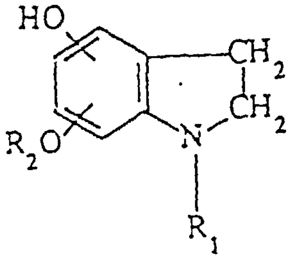

(I)

worin gilt:

$R_1$ stellt ein Wasserstoffatom oder eine $C_{1-4}$-Alkylgruppe dar;
$R_2$ stellt ein Wasserstoffatom oder eine $C_{1-4}$-Alkylgruppe dar,
wobei -OH und -$OR_2$ die Positionen 4, 5, 6 oder 7 des Benzolrings einnehmen, mit der Maßgabe, daß, wenn sie die Positionen 5 und 6 einnehmen, $R_2$ eine $C_{1-4}$-Alkylgruppe bedeutet,

sowie von deren Säureadditionssalzen

als alleinige Farbstoff-Vorstufenverbindung.

2. Verwendung gemäß Anspruch 1,
dadurch **gekennzeichnet**, daß
die Salze aus Hydrochloriden oder Hydrobromiden ausgewählt sind.

3. Verwendung gemäß Anspruch 1,
dadurch **gekennzeichnet**, daß
das Hydroxyindolinderivat aus

- 7-Methoxy-6-hydroxyindol
- 6,7-Dihydroxyindolin
- 5-Hydroxy-4-methoxyindolin
- 4,5-Dihydroxyindolin
- 5-Methoxy-6-hydroxyindolin
- 7-Hydroxy-6-methoxyindolin
- 7-Methoxy-4-hydroxyindolin
- 4-Hydroxy-5-methoxyindolin
- 5-Hydroxy-6-methoxyindolin
- 4,7-Dihydroxyindolin

oder aus deren Säureadditionssalzen ausgewählt ist.

4. Färbezusammensetzung zur Färbung keratinischer Fasern,
dadurch **gekennzeichnet**, daß
sie als alleinige Farbstoff-Vorstufenverbindung, in einem zur Färbung geeigneten Milieu, mindestens ein in jedem der Ansprüche 1 bis 3 definiertes Hydroxyindolinderivat der Formel (I) enthält.

5. Zusammensetzung gemäß Anspruch 4,
dadurch **gekennzeichnet**, daß
das Hydroxyindolinderivat der Formel (I) in der Zusammensetzung in Mengenanteilen von 0,01 bis 8 Gew.% vorhanden ist, bezogen auf das Gesamtgewicht der Zusammensetzung.

6. Zusammensetzung gemäß Anspruch 4 oder 5,
dadurch **gekennzeichnet**, daß
das zur Färbung geeignete Milieu ein wässriges Milieu aus Wasser oder aus einer Mischung aus Wasser/Lösungsmittel(n) ist.

7. Zusammensetzung gemäß Anspruch 6,
dadurch **gekennzeichnet**, daß
die Lösungsmittel aus Ethyl-, Propyl-, Isopropyl- oder t-Butylalkohol, Ethylenglycol, Monomethyl-, Monoethyl- und Monobutylethern von Ethylenglycol, Monoethyletherethylenglycolacetat Propylenglycol, Monomethylethern von Propylenglycol und Dipropylenglycol und aus Methyllactat ausgewählt sind.

8. Zusammensetzung gemäß einem der Ansprüche 4 bis 7,
dadurch **gekennzeichnet**, daß
die Zusammensetzung Fettamide, anionische, kationische, nicht-ionische oder amphotere oberflächenaktive Mittel oder deren Mischungen, Verdickungsmittel, Parfüm-Produkte, Sequestriermittel, filmbildende Mittel, Behandlungsmittel, Dispergiermittel, Konditioniermittel, Konservierungsmittel, opak machende Mittel und Mittel zur Auflockerung keratinischer Fasern oder deren Mischungen enthält.

9. Zusammensetzung gemäß einem der Ansprüche 4 bis 8,
dadurch **gekennzeichnet**, daß
der pH-Wert der Zusammensetzung 3 bis 12 beträgt.

10. Verfahren zur Färbung keratinischer Fasern, insbesondere menschlicher keratinischer Fasern,
dadurch **gekennzeichnet**, daß
man auf diese Fasern mindestens eine in jeden der Ansprüche 4 bis 9 definierte Zusammensetzung aufbringt, man diese Zusammensetzung in Kontakt mit den Fasern über eine Zeitspanne hält, die ausreicht, um eine Färbung zu entwicklen, entweder an der Luft oder mittels eines oxidierenden Systems, und daß man sodann eine Spülung

durchführt.

**11.** Verfahren gemäß Anspruch 10,
dadurch **gekennzeichnet**, daß
sich die Färbung durch Kontakt an der Luft entwickelt, ohne daß ein äußeres oxidierendes Mittel zugegeben wird.

**12.** Verfahren gemäß Anspruch 10,
dadurch **gekennzeichnet**, daß
man auf die Fasern eine Zusammensetzung (A) aufbringt, die in einem zur Färbung geeigneten Milieu mindestens ein in jedem der Ansprüche 1 bis 3 definiertes Hydroxyindolinderivat der Formal (I) enthält, wobei die Farbe mit einem chemischen oxidierenden System entwickelt wird, das zusammengesetzt ist aus:

(i) Jodidionen und Wasserstoffperoxid, wobei die Zusammensetzung (A) das Hydroxyindolinderivat der Formel (I) enthält, wobei die Zusammensetzung in diesem Fall ausserdem entweder Jodidionen oder Wasserstoffperoxid enthält, und wobei die Aufbringung bzw. Anwendung der Zusammensetzung (A) vor oder nach der Aufbringung bzw. Anwendung einer Zusammensetzung (B) erfolgen, die in einem zur Färbung geeigneten Milieu entweder enthält:

(a) Wasserstoffperoxid bei einem pH-Wert von 2 bis 12 und vorzugsweise von 2 bis 7, wenn die Zusammensetzung (A) Jodidionen enthält, oder enthält:

(b) Jodidionen bei einem pH-Wert von 3 bis 11, wenn die Zusammensetzung (A) Wasserstoffperoxid enthält;

(ii) aus Nitriten, wobei die Aufbringung der Zusammensetzung (A), die das Hydroxyindolinderivat der Formel (I) enthält, nach der Aufbringung einer wässrigen Zusammensetzung (B) erfolgt, die einen sauren pH-Wert aufweist, und wobei die Zusammensetzung (A) oder die Zusammensetzung (B) mindestens ein Nitrit enthalten;

(iii) aus oxidierenden Mitteln, ausgewählt aus Wasserstoffperoxid, Perjodsäure und ihren wasserlöslichen Salzen, Natriumhypochlorit, Chloramin T, Chloramin B, Kaliumferricyanid, Silberoxid, Fenton-Reagens, Blei(IV) oxid, Cäsiumsulfat, Ammoniumpersulfat und aus Alkalichloriten, wobei diese oxidierenden Mittel in der Zusammensetzung (A) vorhanden sind, die das Hydroxyindolinderivat der Formel (I) enthält, oder gleichzeitig mit oder im Anschluß an eine Zusammensetzung (B) aufgebracht werden, welche diese in einem zur Färbung geeigneten Milieu enthält;

(iv) aus Metallanionen, ausgewählt aus Permanganaten oder Bichromaten, wobei diese oxidierenden Mittel mit einer wässrigen Zusamensetzung (B), bei einem pH-Wert von 2 bis 10, vor Anwendung der Zusammensetzung (A) aufgebracht werden;

(v) aus Metallsalzen der Gruppen 3 bis 8 des Periodensystems, wobei diese Metallsalze mit einer Zusammensetzung(B) aufgebracht werden, welche diese in einem zur Färbung geeigneten Milieu enthält, und wobei die Zusammensetzung (B) vor oder nach der Anwendung der Zusammensetzung (A) aufgebracht wird;

(vi) aus Salzen seltener Erden, wobei diese Salze seltener Erden mit einer Zusammensetzung (B) aufgebracht werden, welche diese in einem zur Färbung geeigneten Milieu enthält, und wobei die Zusammensetzung (B) vor oder nach der Anwendung der Zusammensetzung (A) aufgebracht wird, die das Hydroxyindolinderivat der Formel (I) enthält;

(vii) aus einem Chinonderivat, ausgewählt aus o- oder p-Benzochinonen, o- oder p-Benzochinonmonoiminen oder -diiminen, 1,2- oder 1,4-Naphthochinonen, o- oder p-Benzochinonsulfonimiden, $\alpha,\omega$-Alkylenbis-1,4-benzochinonen oder 1,2- oder 1,4-Naphthochinonmonoiminen oder -diiminen, und zwar mittels einer Zusammensetzung (B), die diese in einem zur Färbung geeigneten Milieu enthält, wobei das Hydroxyindolinderivat der Formel (I) und die Chinonderivate so ausgewählt sind, daß die Differenz $\delta E$ des Redoxpotentials zwischen dem Redoxpotential Ei des Hydroxyindolinderivats der Formel (I), bestimmt bei pH = 7 in einem Phosphat-Milieu an einer glasartigen Kohlenstoffelektrode durch Voltametrie, und dem Redoxpotential Eq des Chinonderivats, bestimmt bei pH = 7 in einem Phosphat-Milieu durch Polarographie an einer Quecksilberelektrode, bezogen auf eine gesättigte Kalomel-Elektrode, die folgende Gleichung erfüllt:

$$\delta E = Ei - Eq \leq 320 \text{ Millivolt},$$

wobei die Zusammensetzung (B) vor oder nach der Aufbringung der Zusammensetzung (A) angewandt wird, die das Hydroxyindolinderivat der Formel (I) enthält.

**13.** Verfahren gemäß Anspruch 12,
dadurch **gekennzeichnet**, daß
man auf die keratinischen Materien eine Zusammensetzung (A), die in einem zur Färbung geeigneten Milieu mindestens ein Hydroxyindolinderivat der Formel (I) enthält, zusammen mit Jodidionen aufbringt, wobei die Aufbringung der Zusammensetzung (A) vor oder nach der Aufbringung einer Zusammensetzung (B) erfolgt, die in einem zur Färbung geeigneten Milieu Wasserstoffperoxid enthält.

**14.** Verfahren gemäß Anspruch 12,
dadurch **gekennzeichnet**, daß
man auf die keratinischen Fasern mindestens eine Zusammensetzung (A), die in einem zur Färbung geeigneten Milieu ein Hydroxyindolinderivat der Formel (I) enthält, zusammen mit Wasserstoffperoxid aufbringt, das einen pH-Wert von 2 bis 7 aufweist, wobei die Aufbringung der Zusammensetzung (A) vor oder nach der Aufbringung einer Zusammensetzung (B) erfolgt, die in einem zur Färbung geeigneten Milieu Jodidionen enthält.

**15.** Verfahren gemäß Ansoruch 13 oder 14,
dadurch **gekennzeichnet**, daß
die Jodidionen in der Zusammensetzung (A) oder (B) in Mengenanteilen von 0,007 bis 4 Gew.%, ausgedrückt in $J^-$-Ionen, vorhanden sind, bezogen auf das Gesamtgewicht der Zusammensetzung (A) oder (B).

**16.** Verfahren gemäß Anspruch 12,
dadurch **gekennzeichnet**, daß
man auf die keratinischen Materien eine Zusammensetzung (A), die das Hydroxyindolinderivat der Formel (I) enthält, und sodann eine wässrige saure Zusammensetzung (B) aufbringt, wobei die Zusammensetzung (A) oder die Zusammensetzung (B) mindestens ein Nitrit enthalten, ausgewählt aus Alkali-, Erdalkali- oder Ammoniumnitriten oder aus Nitriten eines jeden weiteren kosmetisch geeigneten Kations, aus organischen Nitritderivaten und aus Vektoren von Nitrit, die ein Nitrit das oben definierten Typs erzeugen, wobei diese Nitrite in Mengenanteilen von 0,02 bis 1 Mol/l vorhanden sind.

**17.** Verfahren gemäß Anspruch 12,
dadurch **gekennzeichnet**, daß

man auf die keratinischen Fasern eine Zusammensetzung (B), die in einem zur Färbung ggeeigneten Milieu, bei einem pH-Wert von 2 bis 10, ein Metallanion mit einer guten Affinität für das Keratin enthält, ausgewählt aus Permanganaten oder Bichromaten, und man zu einem zweiten Zeitpunkt eine Zusammensetzung (A) aufbringt, die in einem zur Färbung geeigneten Milieu, bei einem pH-Wert von 4 bis 10, ein Hydroxyindolinderivat der Formel (I) enthält, wobei die Permanganate oder Bichromate in Molalitäten an Anionen von mehr als $10^{-3}$ Mol/1000 g verwendet werden,
und daß die Zusammensetzungen kein organisches Mittel enthalten, das gegenüber den Anionen eine reduzierende Wirkung ausübt.

**18.** Verfahren gemäß Anspruch 12,
dadurch **gekennzeichnet**, daß
man auf die keratinischen Fasern eine Zusammensetzung (A), die in einem zur Färbung geeigneten Milieu ein Hydroxyindolinderivat der Formel (I) enthält, und vor oder nach der Zusammensetzung (A) eine Zusammensetzung (B) aufbringt, die in einem zur Färbung geeigneten Milieu ein Metallsalz enthält, ausgewählt aus Salzen von Mangan, Kobalt, Eisen, Kupfer und Silber, wobei diese Metallsalze in Mengenanteilen von 0,01 bis 2 Gew.%, ausgedrückt in Metallionen, verwendet werden, bezogen auf das Gesamtgewicht der Zusammensetzung.

**19.** Verfahren gemäß Anspruch 12,
dadurch **gekennzeichnet**, daß
man eine Zusammensetzung (A), die in einem zur Färbung geeigneten Milieu mindestens ein Hydroxyindolinderivat der Formel (I) enthält, und vor oder nach dieser Zusammensetzung eine Zusammensetzung (B) aufbringt,

die in einem zur Färbung geeigneten Milieu ein Salz seltener Erden enthält, ausgewählt aus den Salzen von Cer, Lanthan, Europium, Gadolinium, Ytterbium und Dysprosium, wobei diese Salze seltener Erden in Mengenanteilen von 0,1 bis 8 Gew.% vorhanden sind, bezogen auf das Gesamtgewicht der Zusammensetzung.

**20.** Verfahren gemäß Anspruch 12,
dadurch **gekennzeichnet**, daß
man eine Zusammensetzung (A), die in einem zur Färbung geeigneten Milieu mindestens ein Hydroxyindolinderivat der Formel (I) enthält, und vor oder nach dieser Zusammensetzung eine Zusammensetzung (B) aufbringt, die in einem zur Färbung geeigneten Milieu ein Chinonderivat enthält, ausgewählt aus 1,4-Benzochinon und aus 2-β-Hydroxyethylthio-1,4-benzochinon, wobei dieses Chinonderivat in Mengenanteilen von 0,005 bis 1 Mol/l vorhanden ist.

**21.** Verfahren gemäß einem der Ansprüche 12 bis 15,
dadurch **gekennzeichnet**, daß
man als oxidierendes Mittel eine Zusammensetzung auf Basis von Wasserstoffperoxid verwendet, wobei der Gehalt an Wasserstoffperoxid in der Zusammensetzung 1 bis 40 Volumina beträgt.

**22.** Mittel zur Färbung keratinischer Fasern, insbesondere menschlicher keratinischer Fasern, aus mehreren Bestandteilen,
dadurch **gekennzeichnet**, daß
es einen ersten Bestandteil aus einer in jedem der Ansprüche 12 bis 20 definierten Zusammensetzung (A), enthaltend ein Hydroxyindolinderivat der Formel (I), und einen zweiten Bestandteil aus einer der in jedem der Ansprüche 12 bis 21 definierten Zusammensetzungen (B) umfaßt.

**23.** Vorrichtung aus mehreren Teilstücken oder "Kit zur Färbung",
dadurch **gekennzeichnet**, daß
diese verschiedenen Teilstücke die verschiedenen Bestandteile des in Anspruch 22 definierten Mittels zur Färbung enthalten.

**24.** Verbindungen, die aus den Hydrobromiden der in jedem der Ansprüche 1 bis 3 definierten Hydroxyindolinderivate der Formel (I) zusammengesetzt sind.

**25.** 6-Hydroxy-7-methoxyindolin und seine Säureadditionssalze.

**26.** 6,7-Dihydroxyindolin und seine Säureadditionssalze.

**27.** Zusammensetzung gemäß Anspruch 5,
dadurch **gekennzeichnet**, daß
das Hydroxyindolinderivat der Formel (I) in der Zusammensetzung in Mengenanteilen von 0,03 bis 5 Gew.% vorhanden ist, bezogen auf das Gesamtgewicht der Zusammensetzung.

**28.** Verfahren gemäß Anspruch 17,
dadurch **gekennzeichnet**, daß
die Permanganate oder Bichromate in Molalitäten an Anion bis 1 Mol pro 1000 g verwendet werden.

**Claims**

**1.** Use for dyeing keratinous fibres, in particular human keratinous fibres, of a hydroxyindoline derivative of formula:

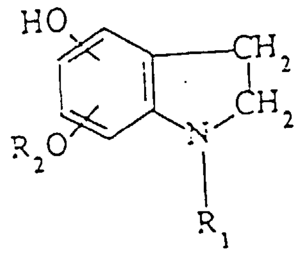

(I)

where:

R1 represents a hydrogen atom or a C1-C4 alkyl radical;
R2 represents a hydrogen atom or a C1-C4 alkyl radical;
OH and -OR2 are in the 4, 5, 6 or 7 positions of the benzene ring, provided that, when they are in the 5 and 6 positions, R2 denotes a C1-C4 alkyl;
and addition salts thereof with acids, as sole dye precursor.

**2.** Use according to Claim 1, characterized in that the salts are selected from hydrochlorides or hydrobromides.

**3.** Use according to Claim 1, characterized in that the hydroxyindoline derivative is selected from 7-methoxy-6-hydroxyindoline, 6,7-dihydroxyindoline, 5-hydroxy-4-methoxyindoline, 4,5-dihydroxyindoline, 5-methoxy-6-hydroxyindoline, 7-hydroxy-6-methoxyindoline, 7-methoxy-4-hydroxyindoline, 4-hydroxy-5-methoxyindoline, 5-hydroxy-6-methoxyindoline, and 4,7-dihydroxyindoline or addition salts thereof with acids.

**4.** Dye composition intended for use in dyeing keratinous fibres, characterized in that it contains, as sole dye precursor, at least one hydroxyindoline derivative of formula (I) as defined in any one of Claims 1 to 3 in an appropriate medium for dyeing.

**5.** Composition according to Claim 4, characterized in that the hydroxyindoline derivative of formula (I) is present in the composition in proportions of between 0.01 and 8% by weight with respect to the total weight of the composition.

**6.** Composition according to Claim 4 or 5, characterized in that the appropriate medium for dyeing is an aqueous medium consisting of water or a water/solvent(s) mixture.

**7.** Composition according to Claim 6, characterized in that the solvents are selected from ethyl alcohol, propyl or isopropyl alcohol, tert-butyl alcohol, ethylene glycol, monomethyl, monoethyl and monobutyl ethers of ethylene glycol, ethylene glycol monoethyl ether acetate, propylene glycol, propylene glycol and dipropylene glycol monomethyl ethers and methyl lactate.

**8.** Composition according to any one of Claims 4 to 7, characterized in that the composition contains fatty amides, anionic, cationic, non-ionic or amphoteric surfactants or mixtures thereof, thickening agents, perfumes, sequestering agents, film-forming agents, treatment agents, dispersing agents, packaging agents, preservatives, opacifiers and agents for swelling keratinous fibres or mixtures thereof.

**9.** Composition according to any one of Claims 4 to 8, characterized in that the pH of the composition is between 3 and 12.

**10.** Method of dyeing keratinous fibres, in particular human keratinous fibres, characterized in that at least one composition as defined in any one of Claims 4 to 9 is applied to those fibres, in that this composition is left in contact with the fibres for a sufficient time for a colour to develop, either in air or using an oxidizing system, and in that rinsing is then carried out.

**11.** Method according to Claim 10, characterized in that the colour is allowed to develop in contact with air without the addition of an external oxidizing agent.

**12.** Method according to Claim 10, characterized in that a composition (A) containing, in an appropriate medium for

dyeing, at least one hydroxyindoline derivative of formula (I) as defined in any one of Claims 1 to 3 is applied to the fibres, the colour being developed using a chemical oxidizing system consisting of:

(i) iodide ions and hydrogen peroxide, composition (A) additionally containing in this case either (a) iodide ions or (b) hydrogen peroxide, and the application of composition (A) being preceded or followed by the application of a composition (B) containing, in an appropriate medium for dyeing, either:

(a) hydrogen peroxide at a pH of between 2 and 12 when composition (A) contains iodide ions, or:
(b) iodide ions at a pH of between 3 and 11 when composition (A) contains hydrogen peroxide;

(in) nitrites, the application of composition (A) being followed by the application of an aqueous composition (B) having an acidic pH, composition (A) or composition (B) containing at least one nitrite;
(iii) oxidizing agents selected from hydrogen peroxide, periodic acid and its water-soluble salts, sodium hypochlorite, chloramine T, chloramine B, potassium ferricyanide, silver oxide, Fenton's reagent, lead(IV) oxide, caesium sulphate, ammonium persulphate and alkaline chlorites; these oxidizing agents being present in composition (A) containing the hydroxyindoline derivative of formula (I) or applied simultaneously or sequentially by means of a composition (B) containing them in an appropriate medium for dyeing;
(iv) metal anions selected from permanganates or dichromates, these oxidizing agents being applied by means of an aqueous composition (B), at a pH of 2 to 10, before the application of composition (A);
(v) salts of metals from Groups 3 to 8 of the Periodic Table, these metal salts being applied by means of a composition (B) containing them in an appropriate medium for dyeing, composition (B) being applied before or after the application of composition (A);
(vi) rare earth salts, these rare earth salts being applied by means of a composition (B) containing them in an appropriate medium for dyeing, composition (B) being applied before or after the application of composition (A) containing the hydroxyindoline derivative of formula (I);
(vii) a quinone derivative selected from ortho- or para-benzoquinones, ortho- or para-benzoquinone monoimines or diimines, 1,2- or 1,4-naphthoquinones, ortho- or para-benzoquinone sulphonimides, $\alpha,\omega$-alkylene-bis-1,4-benzoquinones or 1,2 or 1,4-naphthoquinone monoimines or diimines by means of a composition (B) containing them in an appropriate medium for dyeing, the hydroxyindoline derivative of formula (I) and the quinone derivatives being selected so that the difference in redox potential $\Delta E$ between the redox potential Ei of the hydroxyindoline derivative of formula (I) determined voltametrically at pH 7 in a phosphate medium on a vitreous carbon electrode and the redox potential Eq of the quinone derivative determined polarographically at pH 7 in a phosphate medium on a mercury electrode with reference to the saturated calomel electrode is such that:

$$\Delta E = Ei - Eq \leq 320 \text{ millivolts;}$$

composition (B) being applied before or after the application of composition (A) containing the hydroxyindoline derivative of formula (I).

**13.** Method according to Claim 12, characterized in that a composition (A) containing, in an appropriate medium for dyeing, at least one hydroxyindoline derivative of formula (I) in combination with iodide ions is applied to the keratinous materials, the application of composition (A) being preceded or followed by the application of a composition (B) containing hydrogen peroxide in an appropriate medium for dyeing.

**14.** Method according to Claim 12, characterized in that at least one composition (A) containing a hydroxyindoline derivative of formula (I) in combination with hydrogen peroxide in an appropriate medium for dyeing and having a pH of between 2 and 7 is applied to the keratinous fibres, the application of composition (A) being preceded or followed by the application of a composition (B) containing iodide ions in an appropriate medium for dyeing.

**15.** Method according to Claim 13 or 14, characterized in that the iodide ions are present in composition (A) or (B) in proportions of between 0.007 and 4% by weight, expressed in terms of $I^-$ ions, with respect to the total weight of composition (A) or (B).

**16.** Method according to Claim 12, characterized in that a composition (A) containing the hydroxyindoline derivative of formula (I) is applied to the keratinous materials and in that an aqueous acidic composition (B) is applied next, composition (A) or composition (B) containing at least one nitrite selected from nitrites of alkali or alkaline-earth

metals or ammonium or of any other cosmetically acceptable cation, an organic nitrite derivative and nitrite vectors generating a nitrite of the type defined above, these nitrites being present in proportions of between 0.02 and 1 mol/litre.

**17.** Method according to Claim 12, characterized in that a composition (B) containing, in an appropriate medium for dyeing, at a pH of between 2 and 10, an anion of a metal having good affinity for keratin selected from permanganates or dichromates is applied to the keratinous fibres, and in that a composition (A) is applied which contains a hydroxyindoline derivative corresponding to the formula (I) in an appropriate medium for dyeing, at a pH of between 4 and 10, these permanganates or dichromates being used in anion molalities greater than $10^{-3}$ mol/1000 g,
and in that the compositions do not contain any organic agent having a reducing effect on the anions.

**18.** Method according to Claim 12, characterized in that a composition (A) containing a hydroxyindoline derivative of formula (I) in an appropriate medium for dyeing is applied to the keratinous fibres, and in that, before or after composition (A), a composition (B) is applied containing a metal salt selected from manganese, cobalt, iron, copper and silver salts in an appropriate medium for dyeing, these metal salts being used in proportions of between 0.01 and 2% by weight, expressed in terms of metal ions, with respect to the total weight of the composition.

**19.** Method according to Claim 12, characterized in that a composition (A) containing at least one hydroxyindoline derivative of formula (I) is applied in an appropriate medium for dyeing, and a composition (B) containing a rare earth salt selected from cerium, lanthanum, europium, gadolinium, ytterbium and dysprosium salts in an appropriate medium for dyeing is applied before or after composition (A), these rare earth salts being present in proportions of between 0.1 and 8% by weight with respect to the total weight of the composition.

**20.** Method according to Claim 12, characterized in that a composition (A) containing at least one hydroxyindoline derivative of formula (I) in an appropriate medium for dyeing is applied and in that a composition (B) containing a quinone derivative selected from 1,4-benzoquinone and 2-β-hydroxyethylthio-1,4-benzoquinone in an appropriate medium for dyeing is applied before or after composition (A), this quinone derivative being present in proportions of between 0.005 and 1 mol/litre.

**21.** Method according to Claims 12 to 15, characterized in that when a hydrogen peroxide-based composition is used as oxidizing means, the hydrogen peroxide content of the composition is between 1 and 40 volumes.

**22.** Agent for dyeing keratinous fibres, in particular human keratinous fibres, with a plurality of components, characterized in that it comprises a first component consisting of a composition (A) containing a hydroxyindoline derivative of formula (I) defined in any one of Claims 12 to 20, and a second component consisting of one of the compositions (B) defined in any one of Claims 12 to 21.

**23.** Device with a plurality of compartments, or a "dyeing kit", characterized in that these various compartments contain the various components of the dyeing agent defined in Claim 22.

**24.** Compounds constituted by the hydrobromides of hydroxyindoline derivatives of formula (I) as defined in any one of Claims 1 to 3.

**25.** 6-Hydroxy-7-methoxyindoline and its addition salts with acids.

**26.** 6,7-Dihydroxyindoline and its addition salts with acids.

**27.** Composition according to Claim 5, characterized in that the hydroxyindoline derivative of formula (I) is present in the composition in proportions of between 0.03 and 5% by weight with respect to the total weight of the composition.

**28.** Method according to Claim 17, characterized in that the permanganates or dichromates are used in anionic-molalities ranging up to one mole per 1000 g.